# EUROPEAN PATENT APPLICATION

(11) **EP 4 035 738 A1**
(43) Date of publication of application: **03.08.2022**
(21) Application number: 20868914.1
(22) Date of filing: 18.09.2020
(51) Int. Cl.: A61Q 17/04, A61K 8/24

(54) **NEAR-INFRARED SCREENING AGENT AND NEAR-INFRARED SCREENING EXTERNAL PREPARATION FOR SKIN**

(30) Priority: 27.09.2019 JP 2019176978
(71) Applicant: TOYO BEAUTY CO., LTD., Osaka-shi, Osaka 537-0021 (JP)
(72) Inventor: YAMAMOTO Chiaki, Osaka-shi, Osaka 537-0021 (JP); HISAMA Sanae, Osaka-shi, Osaka 537-0021 (JP); HISAMA Masayoshi, Osaka-shi, Osaka 537-0021 (JP); YOSHIO Kimio, Osaka-shi, Osaka 537-0021 (JP)
(74) Representative: Grünecker Patent- und Rechtsanwälte PartG mbB
(86) International application number: PCT/JP2020/035443
(87) International publication number: WO 2021/060185

(57) **Abstract**

A near infrared blocking agent is provided which contains hydroxyapatite as an active ingredient effective to block near infrared rays. Also provided is a near infrared blocking topical skin preparation of which the content of the hydroxyapatite as the active ingredient is 0.1 to 20% by mass.

## Description

### TECHNICAL FIELD

This invention relates to a near infrared blocking agent and a near infrared blocking topical skin preparation for use in near infrared protection.

### BACKGROUND ART

Near infrared rays, which have long wavelengths (about 800 to 2500 nm), account for, in terms of energy, about half of the solar rays, and easily penetrate the skin, thus dubbed the (biological) "optical window".

It is known that near infrared rays penetrate deep into the skin, and are absorbed into hemoglobin and water in the skin tissue. Taking advantage of this property, near infrared rays are used for medical infrared irradiating devices for improving blood circulation, medical tools, therapeutic devices, vein authentication sensors, optical coherence tomographic diagnostic devices, heaters for household use, fruit sorting machines, and for various other purposes.

Of the solar rays, ultraviolet rays are known to cause skin disorders, including sunburns, formation of stains and wrinkles, and skin cancers, but the energy of the ultraviolet rays accounts for only about 6% of the energy of the solar rays.

In contrast, the energy of the near infrared rays accounts for about half of the energy of the solar rays, and also, they can reach deep into the skin or into the eyes. Thus, there is a concern about various adverse effects of the near infrared rays in the sunlight on living organisms.

For example, it is reported (in the below-identified Non-patent Documents 1 and 2) that 65% or more of the near infrared rays of the sunlight reach the dermis and is absorbed in the dermis to produce heat, and mitochondria in dermal fibroblasts absorbs the near infrared rays and produces reactive oxygen species, which in turn increases the expression of collagen degrading enzyme (MMP-1).

In addition, adverse effects of near infrared rays on living organisms have been reported. For example, it is reported that near infrared rays promote changes in elastin fiber structure and inflammatory reactions in the human dermis (below-identified Non-patent Document 3), and that prolonged exposure can cause skin disorders such as erythema, blistering, and thickening of the skin (in the below-identified Non-patent Document 4).

It is desired to develop inhibitors effective to prevent skin cells from being damaged by near infrared rays, which could have adverse effects on living organisms.

Near infrared shielding materials used in cosmetics include composite materials of inorganic powders of, e.g., titanium dioxide, cerium oxide, talc, and aluminum oxide (below-identified Patent Documents 1 to 3).

### PRIOR ART DOCUMENTS

### PATENT DOCUMENTS

Patent Document 1: JP2006-151916A
Patent Document 2: JP2013-511515A
Patent Document 3: JP2015-86157A

### NON-PATENT DOCUMENTS

Non-patent Document 1: Journal of Investigative Dermatology Symposium Proceedings, 2009, Vol. 14, No. 1, pp. 44-49
Non-patent Document 2: Journal of Investigative Dermatology, 2008, Vol. 128, pp. 2491-2497
Non-Patent Document 3: Journal of Investigative Dermatology Symposium Proceedings, 2009, Vol. 14, No. 1, pp. 15-19
Non-patent document 4: Photodermatol Photoimmunol Photomed, 2003, Vol. 19, pp. 228-234

### SUMMARY OF THE INVENTION

### OBJECT OF THE INVENTION

However, the conventional near infrared shielding materials consisting of inorganic powders described above cannot be said to be sufficiently safe and biocompatible with living organisms, because they may cause allergies when they come into contact with fine wounds formed on the skin.

In addition, it is not easy to stably and uniformly disperse conventional near infrared shielding substances or near infrared blocking agents consisting of inorganic powders in base materials such as cosmetics, or to evenly apply them to the skin surface even if they are dispersed, and for this reason, it is technically difficult to stably demonstrate the near infrared blocking effect with high addition efficiency.

Hydroxyapatite, which was not known to have near infrared blocking properties, has been used for artificial bones and dental cements because it is known to have biocompatibility, and it could have been used as a base material for cosmetics, but it was not efficiently used for the specific application of near infrared blocking properties.

An object of the present invention is to provide, as a new application of hydroxyapatite, to provide a near infrared blocking agent that improves the shortcomings of the conventional near infrared blocking substances, and to provide a near infrared topical skin preparation which is, by using this near infrared blocking agent, sufficiently safe to living organisms, in which the near infrared blocking agent is dispersed such that the near infrared blocking ability will be uniform, and which can cover the skin surface in a spotless manner, thereby efficiently protecting the skin from near infrared rays.

### MEANS FOR ACHIEVING THE OBJECT

In order to achieve this object, the present invention provides a near infrared blocking agent comprising hydroxyapatite as an active ingredient effective to block near infrared rays.

The near infrared blocking agent contains hydroxyapatite, which is highly biocompatible, in a concentration required to provide a required near infrared blocking effect. For example, the content of the hydroxyapatite may be 100% by mass.

By using hydroxyapatite having a plate-shaped or laminar, granular crystal structure, the near infrared blocking agent can be evenly applied to the skin surface while being dispersed in a base material such as water, emulsion or gel, without lowering the biocompatibility of the near infrared blocking agent.

By using hydroxyapatite having a granular crystal structure having an average granule size of 5 to 20 µm, the near infrared blocking agent can be evenly applied to the skin surface, even if its content is increased to, for example, 10 to 20% by mass or over.

A near infrared blocking topical skin preparation according to the present invention contains hydroxyapatite as described above as its active ingredient. The hydroxyapatite is sufficiently safe to living organisms, dispersed such that its infrared blocking performance is uniform, and can be easily applied to the skin surface in a spotless manner.

In order that the near infrared blocking effect is more uniformly revealed, the content of the hydroxyapatite as the active ingredient is preferably 0.1 to 20% by mass of the near infrared blocking topical skin preparation.

Because the hydroxyapatite does not inhibit the effect of other inorganic or organic near infrared blocking agents., the near infrared blocking topical skin preparation may contain, in addition to the near infrared blocking agent, one or both of an ultraviolet scattering agent and an ultraviolet absorbing agent.

### ADVANTAGES OF THE INVENTION

According to the present invention, because the near infrared blocking topical skin preparation uses a near infrared blocking agent containing hydroxyapatite as its active ingredient, it is sufficiently safe to living organisms, and the hydroxyapatite is dispersed such that its infrared blocking performance is uniform, and can be easily applied to the skin surface in a spotless manner. Thus, the near infrared blocking topical skin preparation is capable of efficiently protect the skin against near infrared rays.

### BRIEF DESCRIPTION OF THE DRAWINGS

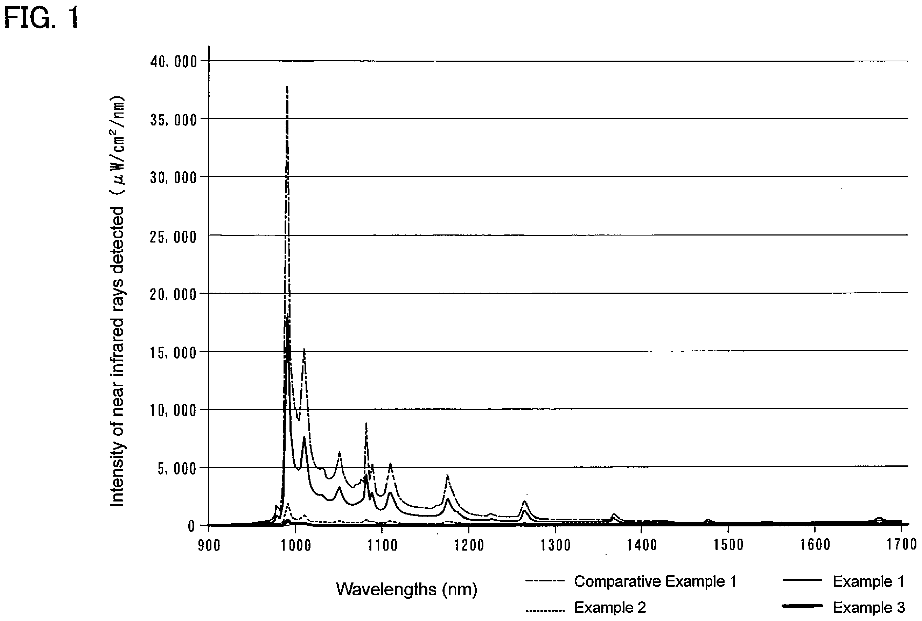

Fig. 1 is a graph illustrating the relationships between the intensities and the wavelengths of near infrared rays for the respective near infrared blocking topical skin preparations of Examples 1 to 3 and Comparative Example 1.

### EMBODIMENT

The near infrared blocking agent and the near infrared blocking topical skin preparation that embody the present invention are now described referring to specific examples.

The near infrared blocking agent according to the present invention contains hydroxyapatite as its active ingredient for near infrared blocking properties. The near infrared blocking agent may be prepared by adjusting the hydroxyapatite concentration so as to obtain a required near infrared blocking properties according to its intended use. For example, the hydroxyapatite concentration may be adjusted within the range of 0.1 to 100% by mass.

Hydroxyapatite, used in the present invention, which is abbreviated as "HAP", is expressed by chemical formula: Ca₁₀(PO₄)₆(OH)₂. Naturally occurring HAP includes a kind of apatite mainly containing hydroxy groups as monovalent anions. It is well known that hydroxyapatite is capable of maintaining its crystal structure even after partial substitution of the ions in the crystal, and thus, by finely adjusting the substitution process, the hydroxyapatite can have a hexagonal column-shaped, plate-shaped, or laminar crystal structure.

The present invention contemplates utilizing the near infrared blocking properties of hydroxyapatite, and using hydroxyapatite, a kind of calcium phosphates, instead of metal particles of e.g., titanium oxide or zinc oxide, which are known to be capable of reflecting near infrared rays, i.e., reflecting, without transmitting, near infrared rays.

Hydroxyapatite is not only widely used in medical fields such as orthopedics and dentistry because it is the main component of teeth and bones and is safe for living organisms, but it can also be used in cosmetics and industrial products. Therefore, when a near infrared-blocking topical skin preparation containing the above near infrared blocking agent as an active ingredient is applied to the skin by, for example, application, spraying, soaking, etc., the surface of the skin is thoroughly covered by the above topical skin preparation due to its biocompatibility, and thus near infrared rays, which could otherwise penetrate deep into the skin, can be efficiently blocked.

The content of the hydroxyapatite as the ingredient effective to block near infrared rays in the near infrared ray blocking topical skin preparation of this invention is 0.1% by mass or more, and for practical reasons, 20% by mass or less. In view of the fact that, as is apparent from the below-described evaluation of the examples, the far infrared blocking efficiency tends to increase sharply when the content is 0.5% by mass or higher, and considering the addition efficiency of the active ingredient, the preferred content of the hydroxyapatite is 0.5% by mass or more and 10% by mass or less.

As long as the topical skin preparation of this invention contains the above-mentioned blocking ingredient, it may contain other ingredients, such as an ultraviolet scattering agent and/or an ultraviolet absorbing agent.

The inclusion of an ultraviolet scattering agent and/or an ultraviolet absorbing agent is preferable because the topical skin preparation is now capable of reducing the adverse effects of not only near infrared rays but also ultraviolet rays, making the topical skin preparation particularly suitable for protecting the skin.

The types of the ultraviolet scattering agent and the ultraviolet absorbing agent may be well-known ones used in the field of topical skin care products such as cosmetics.

Specific examples of ultraviolet scattering agents usable in the invention include inorganic compounds such as zinc oxide and titanium oxide.

Ultraviolet absorbing agents usable in the invention include cinnamic acid compounds, benzophenone compounds, benzoic acid compounds, salicylic acid compounds, dibenzoylmethane compounds, anthranilic acid compounds, and urocanic acid compounds. More specifically, ultraviolet absorbing agents usable in the invention include:
cinnamate compounds such as para-methoxycinnamate-2-ethylhexyl, isopropyl para-methoxycinnamate, para-methoxyhydrocinnamate diethanolamine salt, diparamethoxycinnamate-mono-2-ethylhexanoate glyceryl, octyl methoxycinnamate, and methyl diisopropyl cinnamate;
benzophenone ultraviolet absorbers such as 2-hydroxy-4-methoxybenzophenone, 2-hydroxy-4-methoxybenzophenone-5-sulfate, 2-hydroxy-4-methoxybenzophenone-5-sodium sulfate, 2,4-dihydroxybenzophenone, 2,2'-dihydroxy-4,4'-dimethoxybenzophenone, 2,2'-dihydroxy-4-methoxybenzophenone, 2,2',4,4'-tetrahydroxybenzophenone, and 2-hydroxy-4-n-octoxybenzophenone;
benzoic acid compounds such as para-aminobenzoic acid, ethyl para-aminobenzoate, butyl para-aminobenzoate, 2-ethylhexyl para-dimethylaminobenzoate, glyceryl para-aminobenzoate, octyl para-dimethylaminobenzoate, and amyl para-aminobenzoate;
salicylic compounds such as 2-ethylhexyl salicylate, triethanolamine salicylate, homomenthyl salicylate, dipropylene glycol salicylate, methyl salicylate, ethylene glycol salicylate, phenyl salicylate, amyl salicylate, benzyl salicylate, isopropyl benzyl salicylate, and potassium salicylate;
dibenzoylmethane compounds such as 4-tert-butyl-4'-methoxydibenzoylmethane, 4-isopropyldibenzoylmethane, 4-methoxydibenzoylmethane, and 4-tert-butyl-4'-methoxydibenzoylmethane;
menthyl-O-aminobenzoate;
2-phenyl-benzimidazol-5-sulfate;
2-phenyl-5-methylbenzoxazole;
3-(4-methylbenzylidene)camphor;
2-ethylhexyl-2-cyano-3,3-diphenylacrylate;
2-ethyl-2-cyano-3,3'-diphenylacrylate;
2-(2'-hydroxy-5-methylphenyl)benzotriazole;
anthranilic acid compounds such as menthyl anthranilate; and
urocanic acid compounds such as ethyl urocanate.

The content of the ultraviolet scattering agent or ultraviolet absorbing agent described above is usually 0.001% by mass or more, preferably 0.01% by mass or more. The upper limit is 10% by mass or less, preferably 5% by mass or less. When the content of the ultraviolet absorber is within the above range, an excellent ultraviolet absorption ability can be stably demonstrated.

The near infrared-blocking topical skin preparation of the present invention may be provided in various forms such as, for example, liquid, emulsion, cream, solid, paste, gel, lotion, powder, etc., without limitation, and is preferably in the form of a topical skin preparation for pharmaceuticals, quasi-drugs, cosmetics, etc. Examples of such cosmetics include lotions, emulsions, creams, packs, sun screen agents, conditioners and make-up cosmetics such as foundations, white powders, eye shadows, blushes, lipsticks, etc., depending on the intended use.

In addition, the near infrared shielding topical skin preparations of the present invention may use, as necessary, various ingredients known to be normally used in cosmetics, quasi-drugs, pharmaceuticals, and the like. Such ingredients include:
powders such as chalk, talc, fuller's earth, kaolin, starch, rubber, sodium colloidal silica polyacrylate, etc.;
oils or oily substances such as mineral oil, vegetable oil, silicone oil, etc.;
emulsifiers such as sorbitan trioleate, sorbitan tristearate, glycerol monooleate, and polymeric silicone surfactants;
preservatives such as para-hydroxybenzoate esters;
antioxidants such as butyl hydroxytoluene;
wetting agents such as glycerol, sorbitol, 2-pyrrolidone-5-carboxylate, dibutyl phthalate, gelatin, and polyethylene glycol;
buffers such as lactic acids with bases such as triethanolamine or sodium hydroxide; and
skin protectants such as synthetic, animal, or vegetable ceramides;
waxes such as beeswax, ozokerite wax, paraffin wax. Thickeners, activity enhancers, antibacterial agents, colorants, and fragrances may also be added.

The manufacturing method of the near infrared shielding topical skin preparation of the present invention is not particularly limited, and a well-known manufacturing method may be used as appropriate depending on the form of the topical skin preparation. The topical skin preparation of the present invention will be described by way of specific examples, citing examples and comparative examples below. Unless otherwise specified, the numerical values of the formulations in the examples refer to mass-based values.

### EXAMPLES

### [Examples 1 to 6 and Comparative Examples 1 to 7]

According to the formulations in Tables 1 and 2 below, creams of the near infrared blocking topical skin preparations of the present invention were mixed for example, and made in a regular manner to obtain cream formulations, and the following evaluation tests 1 and 2 were conducted. The near infrared blocking agents used in Examples 1 to 6 are all near infrared blocking agents comprising 100% by mass of hydroxyapatite.

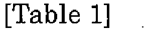

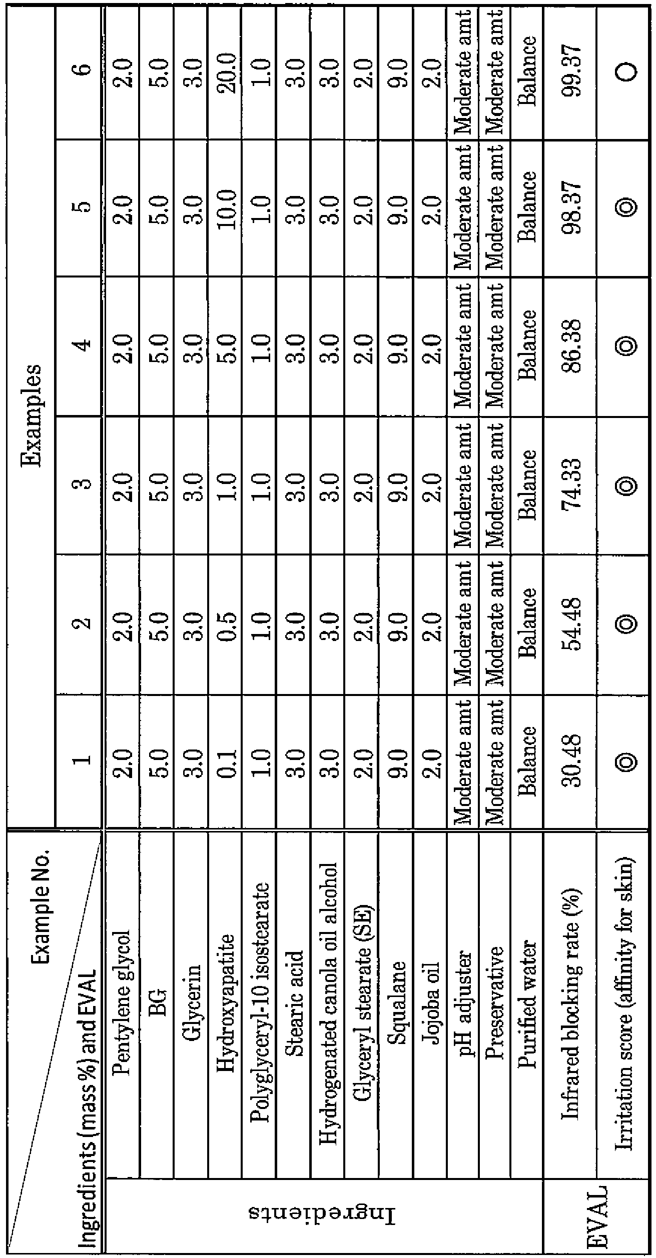

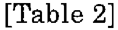

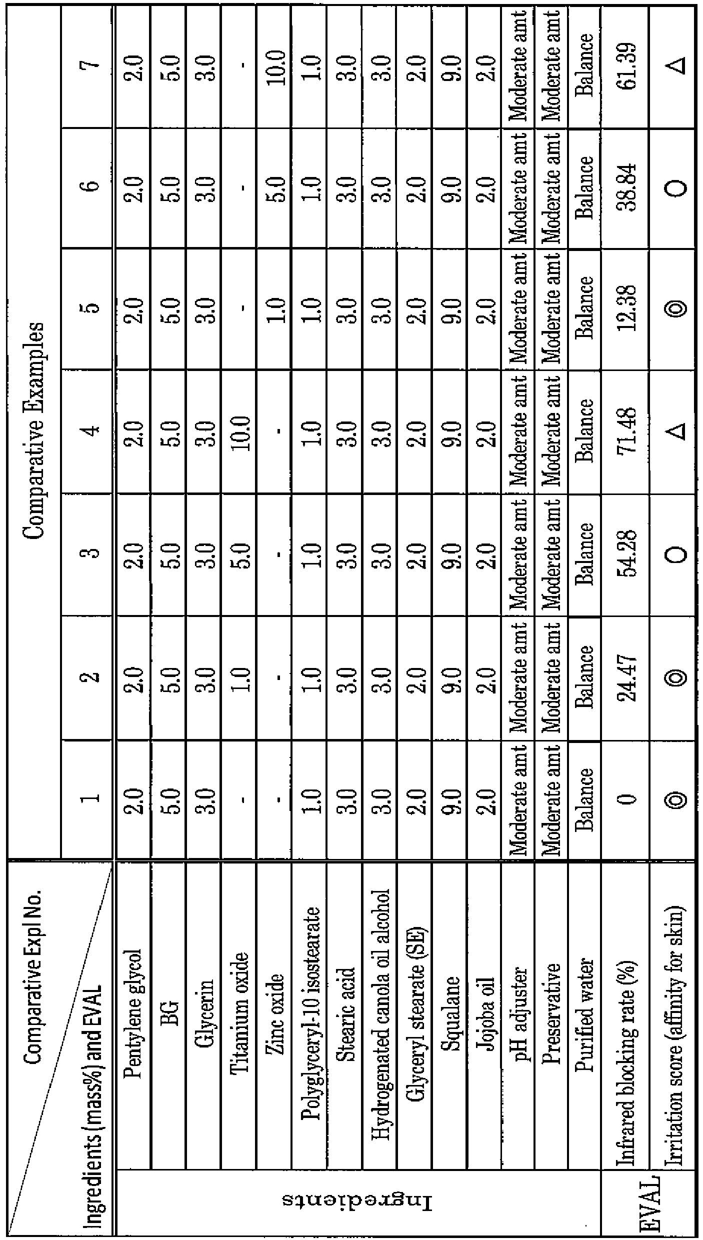

### <Evaluation test 1>

In order to evaluate the near infrared light blocking topical skin preparations (compositions) of the Examples and Comparative Examples, the amount of transmitted light was measured using hydrophilic transparent sheets each attached to a glass slide with a predetermined amount of each of the topical skin preparations spread on the sheet in a thin film. These samples were placed at the terminal, and the light-receiving part measured the intensities of the portion of the near infrared light having wavelengths in the range of 900 nm to 1700 nm, which are the wavelengths that could irradiate the ground without being absorbed or reflected by atmospheric moisture, and thus could affect the skin. The measurement results are shown in the graph (spectra) of Fig. 1, which shows the intensities of the respective wavelengths of the near infrared rays detected after they have passed through each of the samples of Examples 1 to 3 and Comparative Example 1.

The results of Examples 1-3 confirm a decrease, dependent on the concentration of the hydroxyapatite, in detection intensity in the near infrared wavelength region, which was not confirmed in Comparative Example 1. For the portions of the wavelength range of 800-2500 nm other than the wavelength range measured by the light-receiving part, it is presumed that near infrared rays should not be detected, because the detection intensity obtained (pW/cm2/nm) approaches zero at wavelengths of 950 nm or less and 1500 nm or more.

In each of Examples 1 to 6 and Comparative Examples 1 to 7, the intensities of a blank (a sample without a near infrared blocking topical skin preparation) was subtracted from the total intensities, and integrated. Then, from the thus-obtained integrated value of the near infrared intensities, and the integrated value of the near infrared intensities of the blank, the near infrared blocking rate (%) was determined. The results are listed in Tables 1 and 2.

### <Evaluation Test 2>

For ten female subjects between the ages of 20 and 50 years with mildly sensitive skin symptoms, suitable amounts of the topical skin preparations of the Examples and Comparative Examples in Tables 1 and 2 were applied to the face, after cleansing, twice a day (morning and evening) for 2 weeks. After use, the skin appearances and irritation by sensory stimulation (affinity for the skin) were evaluated according to the following standard, and the results are shown in Tables 1 and 2.
- ⊚:: No irritation (0 to less than 20%)
- ○:: Extremely slightly irritating (20 to less than 40%)
- Δ:: Slightly irritating (less than 40-60%)
- ×:: Irritating (more than 60%)

As is apparent from the above results, it was confirmed that Examples 1-6 had significantly higher near infrared ray blocking effect, higher safety, and higher affinity for the skin than titanium dioxide and zinc oxide, which are common light blocking ingredients. Thus, the shortcomings of conventional inorganic near infrared blocking agents for topical skin use were remedied, and the product was safe enough for living organisms, and the near infrared blocking effect was uniformly dispersed and the addition efficiency was good. In addition, from the viewpoint of the addition efficiency of the near infrared blocking agent, it was confirmed that a stable effect was obtained and that it could be easily applied to the skin surface without any spots.

The typical formulations of cosmetics are shown as examples of using a predetermined near infrared blocking ingredient as an active ingredient. In Prescription Examples 1 and 2, purified water was added such that its amount is the balance, that is, the total amount of the listed ingredients will be 100% by mass of the cosmetic.

### [Prescription Example 1] (Emulsion)

Hydroxyapatite: 4.0
1,3-Butylene glycol: 10.0
Carboxyvinyl polymer: 0.3
Squalane: 5.0
Purified hydrogenated soy phospholipid: 0.5
Cetanol: 0.8
L-Arginine: 0.3
Fragrance: moderate amount
Preservative: moderate amount
Purified water: balance

### [Prescription Example 2] (Liquid foundation)

Hydroxyapatite: 15.0
1,3-Butylene glycol: 10.0
Titanium dioxide: 3.0
Zinc oxide: 3.0
Octyl methoxycinnamate: 6.0
Hexyl diethylaminohydroxybenzoyl benzoate 1.0
Carboxyvinyl polymer: 0.3
Stearic acid: 1.0
Behenyl alcohol: 2.0
Hydrogenated polyisobutene: 3.0
Polysorbate 60: 5.0
Potassium hydroxide: 0.2
Fragrance: moderate amount
Preservative: moderate amount
Purified water: balance

### [Prescription Example 3] (Powder foundation)

Hydroxyapatite: 20.0
Hydrophobically treated talc: 31.5
Titanium dioxide: 3.0
Zinc oxide: 3.0
Hydrophobically treated bengara: 1.5
Hydrophobized yellow iron oxide: 3.5
Hydrophobically treated black iron oxide: 0.5
Mica: 10.0
Spherical silica: 10.0
Spherical silicone elastomer: 5.0
Dimethicone: 6.0
Triethylhexanoin: 6.0

## Claims

1. A near infrared blocking agent comprising hydroxyapatite as an active ingredient effective to block near infrared rays.

2. The near infrared blocking agent of claim 1, wherein the hydroxyapatite has a plate-shaped or laminar, granular crystal structure.

3. The near infrared blocking agent of claim 2, wherein the hydroxyapatite has a granular crystal structure having an average granule size of 5 to 20 µm.

4. A near infrared blocking topical skin preparation comprising the near infrared blocking agent of any of claims 1 to 3 as an active ingredient effective to block near infrared rays.

5. The near infrared blocking topical skin preparation of claim 4, wherein the content of the hydroxyapatite as the active ingredient is 0.1 to 20% by mass of the near infrared blocking topical skin preparation.

6. The near infrared blocking topical skin preparation of claim 4 or 5, further comprising, as an additive, one or both of an ultraviolet scattering agent and an ultraviolet absorbing agent.
